# FASCICULE DE BREVET EUROPEEN

(11) **EP 0 506 568 B1**
(45) Date de publication et mention de la délivrance du brevet: **15.02.1995**
(21) Numéro de dépôt: 92400842.8
(22) Date de dépôt: 26.03.1992
(51) Int. Cl.: B08B 9/04, C12M 1/04, F16K 1/00

(54) **Photobioréacteur avec un dispositif de nettoyage automatique et continu de la canalisation du récepteur solaire du photobioréacteur**
Photobioreaktor mit einer Vorrichtung zum automatischen und kontinuierlichen Reinigen der Rohrleitungen des Fotobioreaktorsolarempfängers.
Photobioreactor with a device for the automatic and continuous cleaning of the pipeline in the solar receptor of the photobioreactor

(30) Priorité: 28.03.1991 FR 9103781
(43) Date de publication de la demande: 30.09.1992
(73) Titulaire: HELIOSYNTHESE S.A., 13795 Aix en Provence Cédex 03 (FR)
(72) Inventeur: Chaumont, Daniel, F-13770 Venelles (FR); Ferreira Dos Santos, Patrick, F-13100 Aix en Provence (FR); Sauze, Léopold, F-04300 Saint Maime (FR)
(74) Mandataire: Dubois-Chabert, Guy

(56) Documents cités:
- FR-A- 2 576 034

## Description

La présente invention se rapporte au domaine des photobioréacteurs, c'est-à-dire, des appareils qui permettent la croissance contrôlée de microorganismes photosynthétiques (microalgues...).

De façon classique, les photobioréacteurs comportent en association en série dans une canalisation fermée sur elle-même et parcourue par une solution de milieu nutritif, un carbonateur et un récepteur solaire. Le carbonateur est le point du circuit où la solution est enrichie en gaz carbonique et le récepteur solaire, l'endroit du circuit où la solution nutritive est exposée au rayonnement solaire pour obtenir la transformation du gaz carbonique en biomasse. Cette réaction biochimique correspond à une photopolymérisation du gaz carbonique qui peut être schématisée par l'équation de Myers :

6,14CO₂ + 3,65H₂O + NH₃→C_{6,14}H_{10,3}0_{2,24}N + 6,85O₂

De façon également connue, de tels appareils s'encrassent rapidement si l'on ne prend pas de précautions particulières. En effet, des adhésions de micro algues se produisent naturellement, en particulier sur les parois internes des canalisations du récepteur solaire et l'importance de ce phénomène est fonction de l'espèce d'algues cultivées, ainsi que de la matière constitutive des tubulures composant le récepteur solaire et des conditions de culture. De plus, l'activité de photosynthèse des cellules d'algues peut provoquer l'apparition de poches de gaz à l'intérieur des canalisations du récepteur solaire. Une telle accumulation de gaz se traduit par une réduction du volume de culture exposée à la lumière et diminue ainsi fortement le rendement du système. Elle entraîne également, au niveau de ces poches de gaz, le séchage des micro algues et l'apparition d'une pellicule de cellules mortes. En fonction du débit de circulation qui détermine la vitesse linéaire de la culture dans les tubulures et du type de microorganismes cultivés, des sédimentations peuvent également se produire dans la tubulure. Ces deux effets, (adhésion de micro algues et sédimentation) se traduisent notamment par :
- une diminution du volume de culture exposée à la lumière ;
- une évolution de la culture vers un état hétérogène, car les cellules sédimentées ou fixées sur les parois des tubulures échappent au taux de dilution de la culture continue ;
- une augmentation des risques de contamination par développement de bactéries et/ou de protozoaires qui se développent en l'absence de lumière ou au dépens des cellules mortes.

D'une façon générale, on a déjà utilisé pour nettoyer en permanence l'intérieur des canalisations de tels appareils, l'introduction dans ces tubulures de billes en matière plastique qui sont mises ainsi en circulation avec le milieu de culture et assurent en permanence une agitation du milieu nutritif ainsi qu'un nettoyage des tubulures par frottement des billes sur les parois de ces dernières.

Pour être efficace, ce nettoyage doit être continu et automatique. Par ailleurs, l'utilisation de pompes pour faire circuler la solution nutritive dans les tubulures ainsi que la présence de sondes de mesure, impliquent de limiter le nettoyage par billes au seul récepteur solaire, à l'exclusion du carbonateur. De ce fait, un dispositif de nettoyage d'un tel photobioréacteur doit contenir un système de recyclage des billes entre l'entrée et la sortie du récepteur solaire.

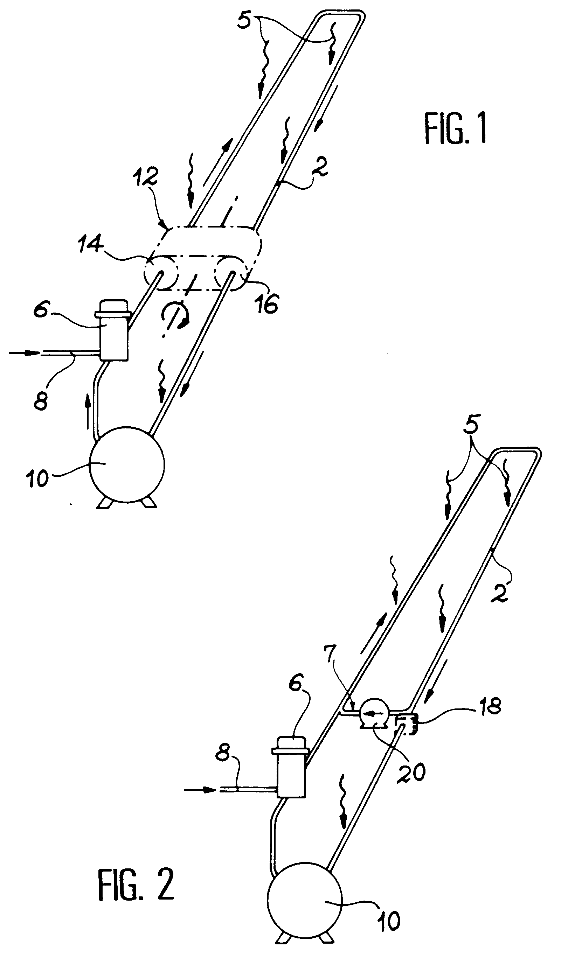

L'art antérieur le plus proche dans ce domaine est représenté par le brevet français FR-A-8500655 qui décrit deux modes de mise en oeuvre pour assurer un tel recyclage. On va décrire ci-après en se référant aux figures 1 et 2, ces deux modes de mise en oeuvre de l'art connu, décrits par le brevet précédemment cité de mise en circulation, des billes de nettoyage dans le seul récepteur solaire. Sur les figures 1 et 2 on a représenté le photobioréacteur avec sa canalisation bouclée sur elle-même et comportant le récepteur solaire 2 soumis au rayonnement 5 ainsi que le carbonateur 6 situé en série avec la boucle précédente. Une pompe 10 assure la circulation du milieu nutritif liquide dans l'ensemble de la canalisation.

Dans le premier mode de réalisation de la figure 1, qui est un mode à fonctionnement manuel, on a recours à un panier de récupération des billes 12 qui comporte deux compartiments 14 et 16. Le compartiment 14 est monté sur la branche aller du circuit du photobioréacteur et le compartiment 16 est monté sur la branche retour de ce circuit. Le panier 12 est monté pivotant de 180° de telle manière que, lorsque l'on tourne le panier, les compartiments 14 et 16 sont inversés. Durant le fonctionnement de l'installation, les billes sont retenues dans le compartiment 16 et s'y accumulent. Lorsque le compartiment 16 est plein, on fait pivoter le panier 12 de 180° de manière à inverser la position des compartiments 14 et 16. Les billes qui se trouvent dans le compartiment 16 peuvent alors être réintroduites dans le circuit par la circulation du fluide. L'inversion des deux paniers 14 et 16 peut se faire sans arrêter la circulation de la culture grâce à des dérivations de canalisation non représentées sur la figure. Toutefois, ce système implique l'ouverture de la canalisation générale et par conséquent la mise en contact pendant un certain temps de la culture avec l'extérieur. Il en résulte des risques de contamination en général difficilement admissibles.

Dans le mode de mise en oeuvre de la figure 2, on assure une séparation nette entre le récepteur solaire 2 et la branche restante de canalisation où se situe le carbonateur 6 en réalisant une dérivation 7 entre la sortie et l'entrée du récepteur solaire de la façon suivante : On a monté une grille d'arrêt des billes 18 sur la branche retour du photobioréacteur. Une pompe spéciale de circulation des billes 20 est montée en dérivation sur les branches aller et retour du circuit en amont de la grille 18 par rapport au sens de l'écoulement de la suspension. La pompe 20 est par exemple une pompe à effet vortex. Dans ce cas, il n'est pas nécessaire que la pompe du circuit principal soit une pompe qui permette la circulation des billes. On peut donc utiliser une pompe de type quelconque. Etant donné que le carbonateur désoxygénateur 6 n'est pas traversé par les billes, car n'étant pas situé sur le circuit de la pompe 20, il n'est pas nécessaire qu'il permette la circulation des billes. Il est donc monté directement sur le circuit.

Dans ce système, la pompe de recyclage 20 des billes fonctionne en continu, ce qui conduit à ce que, en même temps que les billes, une partie importante de culture passe directement dans le récepteur solaire sans être carbonatée. Ceci déjà est un inconvénient majeur pour le rendement de la réaction de photosynthèse et en plus, on a constaté que l'utilisation de pompes sur le circuit du récepteur solaire était très nocif vis-à-vis des cellules de certaines espèces de microalgues.

Pour toutes les raisons précédentes, aucun des deux dispositifs de l'art antérieur permettant de limiter la circulation des billes au seul récepteur solaire ne fonctionne donc de manière satisfaisante.

La présente invention a précisément pour objet un photobioréacteur comportant un récepteur solaire associé à un carbonateur qui permet, à l'aide de moyens dont la mise en oeuvre est simple et efficace, de s'affranchir des inconvénients de l'art antérieur tel qu'on vient de le rappeler.

Ce photobioréacteur selon l'invention se caractérise en ce qu'il comprend :
- sur une portion verticale du conduit de dérivation, une vanne motorisée provoquant le passage automatique des billes, à l'exclusion de la solution ;
- un manchon pivotant intercalé dans la canalisation d'entrée du récepteur solaire, en aval de l'embranchement avec le conduit de dérivation, ce manchon servant de support à une vanne manuelle orientable vis-à-vis de la pesanteur et permettant l'introduction ou le retrait des billes de nettoyage dans/hors de la canalisation ;
- deux doigts de gant étanches fixés sur la paroi de la canalisation, équipés chacun d'une tige pénétrante réglable permettant de stopper ou de laisser passer les billes de nettoyage en laissant circuler la solution, et situés :
   a) pour le premier, à l'embranchement de la canalisation d'entrée du carbonateur et du conduit de dérivation ;
   b) pour le second, dans la canalisation d'entrée du récepteur solaire, immédiatement en aval de la vanne manuelle orientable.

Comme on le verra plus en détail lors de la description ultérieure d'un exemple de mise en oeuvre de l'invention, ce dispositif permet de s'affranchir complètement des défauts de l'art antérieur en utilisant, sur une structure à dérivation à l'entrée du récepteur solaire, simultanément des vannes d'un type particulier dont l'une est manuelle et l'autre automatique, mais qui sont aptes dans les deux cas à ne laisser passer que les billes à l'exclusion de la solution liquide. Ces vannes, utilisées en association avec des tiges pénétrantes au travers de doigts de gant étanches fixés sur la paroi de la canalisation, et qui permettent à volonté de fermer ou d'ouvrir le passage aux billes en permettant celui de la solution, conduisent à un dispositif qui répond totalement aux exigences de la pratique.

Selon une première caractéristique très importante de la présente invention, la vanne motorisée et la vanne manuelle orientable ont un obturateur sphérique creusé d'un trou borgne dont les dimensions sont légèrement supérieures à celles d'une bille.

Cette caractéristique selon laquelle l'obturateur sphérique a la forme d'un godet qui permet de loger une bille, permet, par simple retournement de 180° de faire passer la bille au travers de la vanne tout en maintenant l'étanchéité vis-à-vis de la solution nutritive liquide.

Selon une autre caractéristique de l'invention, la vanne motorisée est équipée d'un détecteur de présence de billes qui, dès la chute par gravité d'une bille dans le trou borgne tourné vers le haut en position d'attente, commande la rotation de l'obturateur une première fois de 180° pour amener l'ouverture du trou borgne vers le bas et libérer la bille par gravité dans le conduit de dérivation puis, une deuxième fois de 180°, pour remettre la vanne en position d'attente de la bille suivante, le trou borgne ouvert vers le haut.

Le montage de cette vanne motorisée dans une canalisation verticale surmontant la conduite d'entrée de la culture dans le récepteur solaire, permet en fonctionnant par gravité de recevoir la bille dans son logement lorsque le trou borgne est orienté vers l'amont de la canalisation verticale et de l'introduire dans le récepteur solaire lorsque ce même trou borgne, après une rotation de 180° de l'obturateur sphérique est tourné vers le bas du conduit de dérivation. Après passage d'une bille, l'obturateur sphérique est tourné à nouveau à 180° pour reprendre sa position initiale le trou borgne étant orienté vers le haut dans l'attente de la bille suivante.

Selon une autre caractéristique de l'invention, le manchon pivotant est placé dans une première position, amenant la vanne manuelle en position verticale au-dessus de la canalisation d'entrée dans le récepteur solaire, pour l'introduction d'une bille dans cette canalisation, et dans une deuxième position à 180° de la première, amenant la vanne manuelle en position verticale au-dessous de la canalisation d'entrée dans le récepteur solaire, pour l'extraction d'une bille hors de cette canalisation.

Le second doigt de gant situé immédiatement en aval de la vanne manuelle orientable étant en position d'ouverture pour les billes et le trou borgne de cette vanne manuelle orientable étant tourné vers le haut, une bille neuve peut y être introduite qui par rotation de 180° de l'obturateur sphérique est amenée par gravité à tomber dans la canalisation d'entrée du récepteur solaire où elle est entraîné par le courant du liquide nutritif.

Si au contraire, le manchon pivotant est tourné de façon telle que la vanne manuelle orientable soit au-dessous de la canalisation avec le trou borgne dirigé vers la canalisation d'entrée du récepteur solaire, il suffit de fermer le second doigt de gant pour que la première bille arrive, tombe par gravité dans l'obturateur sphérique. Il suffit alors de faire pivoter de 180° cet obturateur pour obtenir une chute de la bille vers l'extérieur et son extraction du système.

De toute façon, l'invention sera mieux comprise en se référant à la description qui suit d'un exemple de mise en oeuvre du dispositif de nettoyage automatique, description qui sera faite en se référant aux figures 3 à 5 ci-jointes, sur lesquelles :
- la figure 3 est une vue d'ensemble d'un photobioréacteur conforme à l'invention ;
- la figure 4 est un schéma plus détaillé des systèmes de vannes et de doigts de gant montés sur le dispositif au voisinage de la conduite de dérivation à l'entrée du récepteur solaire ;
- la figure 5 montre en détail la réalisation de l'une des vannes à obturateur sphérique de la figure 4.

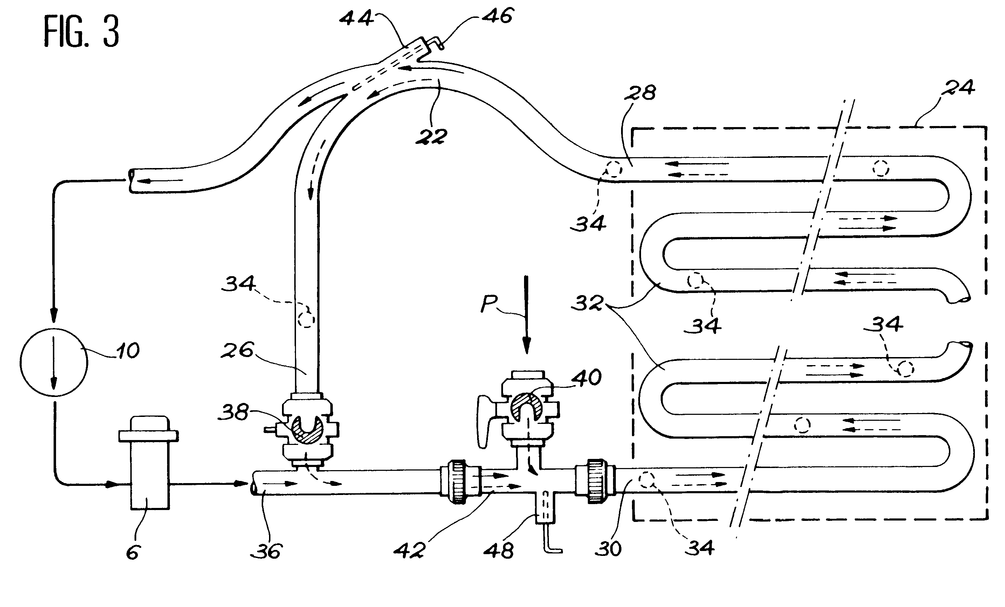

En se référant à la figure 3, qui représente schématiquement vu en plan un photobioréacteur dont la canalisation 22 est fermée selon une boucle parcourant d'une part le récepteur solaire 24 et le circuit du carbonateur du milieu de culture comportant le carbonateur 6 lui-même et la pompe 10, ainsi que le conduit de dérivation 26 qui rejoint la sortie du récepteur solaire 28 à l'entrée 30 de ce même récepteur.

Le récepteur solaire 24 est constitué dans l'exemple décrit d'une série de boucles 32 effectuées par la canalisation 22 et qui sont par définition exposées au rayonnement solaire pour permettre aux microalgues cultivées dans le milieu nutritif qui la parcourent de subir la réaction de photosynthèse chlorophyllienne.

Sur cette figure 3, le trajet de circulation du milieu nutritif liquide est représenté par des flèches en trait plein, alors que le trajet de circulation des billes est représenté par des flèches en pointillé. Dans cet exemple de mise en oeuvre conforme à l'invention, les différentes billes telles que 34 ne traversent que le récepteur solaire 24, le circuit 36 du carbonateur n'étant parcouru que par la solution nutritive liquide. Il est de ce point de vue spécialement intéressant de noter que la pompe 10 qui met en mouvement la circulation de la solution dans toute l'installation est précisément située sur ce trajet 36, c'est-à-dire qu'elle n'est traversée par aucune bille. Cette particularité qui conduit aux avantages du dispositif expliqués précédemment, est rendue possible par la présence sur la dérivation 26 ou autour de celle-ci, des deux vannes à opercule sphérique muni d'un trou borgne en forme de godet dont la première 38 ne laisse passer que les billes et dont la seconde 40 sert à l'introduction ou à l'extraction des billes propres ou destinées au nettoyage. Le bon fonctionnement de ces vannes 38 et 40 implique qu'elles soient, pour la première située sur une partie de canalisation verticale avec un sens de la pesanteur marqué sur la figure par une flèche P et, pour la seconde, montée sur un manchon pivotant 42 permettant de l'orienter verticalement avec l'ouverture de l'obturateur sphérique tourné soit vers le haut, soit vers le bas.

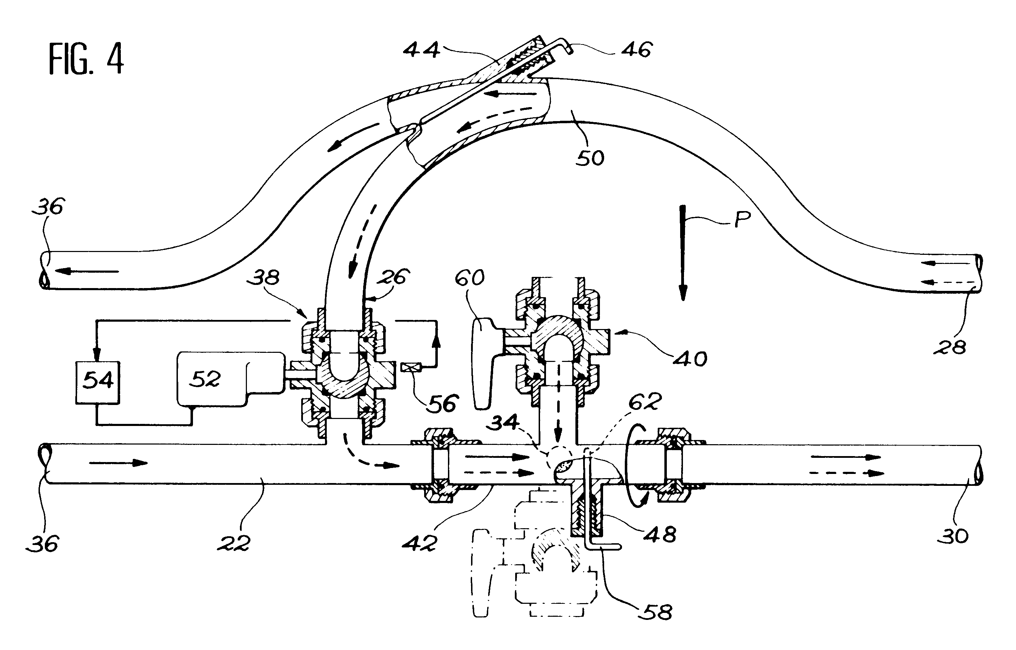

Conformément à l'invention, ces deux vannes sont complétées par la présence de deux doigts de gant étanches munis chacun d'une tige pénétrante réglable et qui permet, soit de stopper, soit de laisser passer les billes de nettoyage, tout en laissant circuler librement la solution. Le premier de ces doigts de gant 44 est situé à l'embranchement de la canalisation du carbonateur 6 et du conduit de dérivation 26. La tige pénétrante 46 peut être maniée manuellement de l'extérieur et permet à volonté d'arrêter les billes 34 en leur interdisant l'entrée dans le circuit du carbonateur 6 lorsque la tige 46 est enfoncée tout en laissant passer la solution nutritive. Le deuxième doigt de gant 48 est situé sur le manchon pivotant 42, immédiatement en aval de la vanne manuelle orientable 40. Dans un mode de mise en oeuvre particulièrement intéressant de l'invention, la vanne 38 est motorisée et comme il sera expliqué plus en détail lors de la description de la figure 4 suivante, commandée automatiquement par une cellule de détection de la présence d'une bille dans la vanne 38.

Dans l'exemple de la figure 3, le récepteur solaire 24 a été représenté comme étant constitué d'un certain nombre de tubes en U 32 dans lesquels circulent plusieurs billes 34 réparties à des intervalles aussi réguliers que possible dans la canalisation. Bien entendu, ceci n'est qu'un exemple et la canalisation du récepteur solaire pourrait avoir tout autre forme et le nombre de billes utilisées dépend du taux d'encrassement de l'installation, le dispositif de nettoyage automatique selon l'invention pouvant fonctionner, à la limite, avec une seule bille. Dans le cas de fonctionnement avec une seule bille, il est important, compte tenu de la durée de révolution de cette bille dans le récepteur solaire 24, de maintenir l'ouverture du trou borgne de l'obturateur sphérique de la vanne 38 vers le bas pour éviter, pendant cette durée, l'encrassement et en particulier la sédimentation dans le trou borgne de l'obturateur sphérique. L'ouverture du trou borgne ne sera réorientée vers le haut, pour recevoir la bille qu'après une temporisation correspondant à la durée de circulation de la bille dans le récepteur solaire 24, ou sur l'ordre d'une cellule de détection placée, par exemple, à la sortie du récepteur solaire.

En se référant maintenant à la figure 4, on va décrire la partie du photobioréacteureur de la figure 3, où se situent les moyens de l'invention, c'est-à-dire la partie située autour de la dérivation 26 de celle-ci.

Sur la figure 4, on a représenté les mêmes éléments que sur la figure 3, les éléments correspondants portant les mêmes nombres de référence.

En ce qui concerne d'abord les différentes conduites ou canalisation, on reconnaît la conduite de dérivation 26 et sur la conduite de circulation générale du milieu nutritif, la partie 36 qui correspond au circuit du système de carbonation et en 28 et 30 respectivement la sortie et l'entrée du récepteur solaire 24 non représenté sur cette figure.

Comme sur la figure 3, le dispositif représenté n'est pas placé indifféremment vis-à-vis de la pesanteur. Celle-ci, est figurée par le vecteur P dans la figure, et, à la sortie du récepteur solaire, la conduite 28 fait une boucle 50 située dans un plan vertical au-dessus de la canalisation 22 pour permettre la chute ou l'introduction par gravité des billes dans la vanne 38.

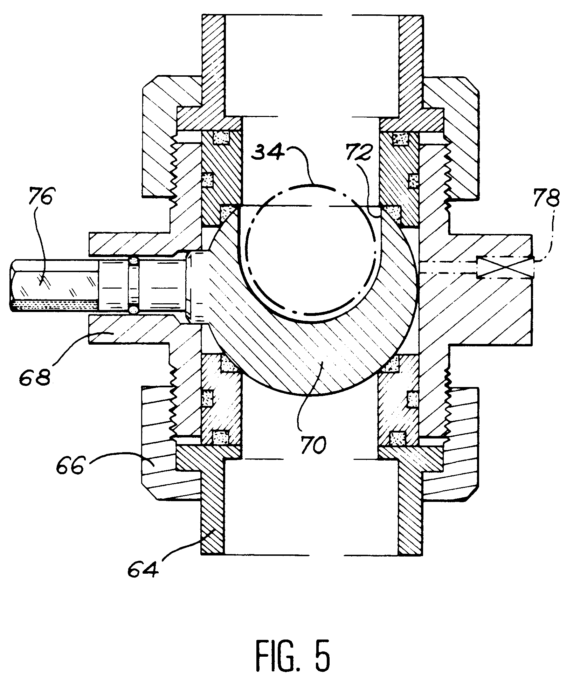

Sur la figure 4, la vanne 38 est manoeuvrée par un moteur 52 qui la fait tourner à chaque fois de 180° sous l'influence d'une commande 54 recevant les indications d'une cellule de détection des billes 56 qui indique la présence d'une de celles-ci dans le trou borgne de l'obturateur sphérique de la vanne 38. La constitution et le fonctionnement de cette vanne 38, ainsi que de la vanne 40, seront mieux compris en complétant la lecture de la description par celle de la figure 5 qui suit maintenant.

En se référant à cette figure 5, on va décrire plus en détail la constitution particulière d'une des vannes manuelles ou motorisées utilisées dans l'invention et référencées 38 et 40 sur la figure 4. Une telle vanne fixée sur la conduite 64 par un écrou 66, comporte un corps de vanne 68 qui sert de logement à l'obturateur sphérique 70 en forme de godet réalisé par creusement d'un trou borgne 72 dans l'obturateur sphérique 70. Ce trou borgne dirigé selon l'axe de la canalisation 64 en position d'ouverture, conduit à donner à l'obturateur de la vanne sa forme caractéristique de godet qui peut, lorsqu'il est orienté comme représenté sur la figure 5 servir de réceptacle à une bille arrivant par la conduite 64, alors qu'il peut au contraire fermer complètement la circulation tant des billes que de la solution liquide arrivant en 64 lorsqu'à l'aide d'une rotation de 180° appliquée par un moteur non représenté à l'axe de commande 76, il présente à la canalisation 64 la partie convexe de l'obturateur 70. On a également représenté pour mémoire sur cette figure 5, le logement 78 d'insertion de la cellule de détection 5 de présence d'une bille dans l'obturateur en forme de godet 70.

Conformément à l'invention, la vanne à obturateur sphérique à commande manuelle 40 est montée sur le manchon pivotant 42 intercalé dans la canalisation d'entrée 30 du récepteur solaire. Ce manchon permet de tourner à la main la vanne 40 pour l'amener en position verticale, soit au-dessus de la canalisation 22 comme c'est le cas sur la figure 4, soit en dessous.

Le doigt de gant 44 a sa tige pénétrante 46 en position d'enfoncement tant que des billes circulent dans la boucle 50, ayant en cela pour effet de laisser la solution nutritive passer vers l'introduction 36 du système de carbonation, tout en contraignant lesdites billes à emprunter la partie descendante verticale de la boucle 26.

Le doigt de gant 46 est situé, comme déjà expliqué, immédiatement en aval de la vanne manuelle 40 sur le manchon 42, à l'entrée dans le récepteur solaire.

Dans ces conditions, le dispositif de la figure 4 fonctionne de la manière suivante.

En position d'attente, c'est-à-dire pendant le temps qui sépare l'arrivée de deux billes consécutives dans la canalisation 28, la vanne motorisée 38 a le trou borgne de son obturateur sphérique tourné vers le haut. Corrélativement et d'une manière générale de façon permanente, le doigt de gant 44 a sa tige pénétrante 46 enfoncée dans l'embranchement qui sépare la boucle 50 de l'entrée 36 du système de carbonation, contraignant ainsi les billes qui pénètrent dans la boucle 50 à passer dans la partie descendante 26 puis à tomber par gravité dans l'obturateur sphérique de la vanne 38. En revanche, comme déjà indiqué, la solution de milieu nutritif contenant la culture d'algues, peut librement passer de la boucle 50 à l'entrée 36 du système de carbonation. Dès qu'une bille est tombée dans le trou borgne de la vanne 38, elle est détectée par une cellule 5 de détection de billes et une information est transmise à la commande 54 du moteur 52 qui effectue alors un demi tour vers le bas. Dans cette position, l'orifice du trou borgne est dirigé vers le bas par rapport à la pesanteur et la bille peut passer librement dans la canalisation 30 dans laquelle elle est entraînée par le milieu nutritif liquide en circulation provenant du retour 36 du sytème de carbonatation. A ce stade, l'absence de la bille dans l'obturateur sphérique de la vanne 38 étant constatée par le détecteur 5, une nouvelle instruction est communiquée par la commande 54 au moteur 52 qui fait à nouveau une rotation de 180° dans un sens ou dans l'autre pour placer l'obturateur sphérique de la vanne 38 à nouveau en position d'attente réceptive de la bille suivante.

La vanne à obturateur sphérique et à commande manuelle 40 permet l'introduction et l'extraction de billes dans le circuit du photobioréacteur.

Les opérations d'introduction se passent de la manière suivante : le trou borgne de l'obturateur étant dirigé vers le haut, une bille neuve est introduite au sommet 56 de cette vanne 40. Corrélativement, la tige 58 du doigt de gant 48 est tiré manuellement vers le bas laissant ainsi libre la circulation d'une bille dans le manchon pivotant 42 et l'obturateur de la vanne 40 est alors tourné manuellement à l'aide de la poignée 60 de 180°. La bille ainsi libérée chute par gravité dans le manchon pivotant 42 et est entraînée dans la circulation générale 30 du photobioréacteur.

Pour les opérations d'extraction d'une bille sale, on tourne par rapport à la représentation de la figure 4, le manchon 42 de 180° pour placer la vanne 40 toujours verticalement, mais en dessous de ce manchon 42. La tige 58 du doigt de gant est alors enfoncée pour stopper la progression éventuelle de la bille suivante dans l'entrée 30 du récepteur solaire et la vanne 40 est placée manuellement par la poignée 60 dans la situation d'avoir le trou borgne de son obturateur situé vers le haut. Dans ces conditions, dès que la bille suivante apparaît dans la canalisation 26, puis dans le manchon 42, elle est bloquée par la tige 58 dans la position représentée en 62 sur la figure 4 puis elle descend par gravité dans la vanne 40 d'où elle peut finalement être extraite par rotation de 180° imprimée à la poignée 60, ce qui place l'entrée du trou borgne de l'obturateur sphérique vers le bas. Elle tombe alors par gravité à l'extérieur où il n'y a plus qu'à la recueillir. On libère alors le passage pour les billes suivantes en tirant la tige pénétrante 58 du doigt de gant 48 pour les remettre dans la position qu'ils occupent sur le dessin de la figure 4. Afin d'éviter toute sédimentation de biomasse, la vanne 40 est remise en position d'attente, c'est-à-dire au-dessus du manchon 42.

Le système 5 de détection de la présence ou de l'absence de billes peut être bien entendu d'une nature quelconque connue. Dans le cas où il est constitué d'une cellule photoélectrique, le corps de la vanne et l'obturateur sphérique en forme de godet doivent être impérativement transparents et on peut les réaliser en matière plastique tel que le polymétacrylate par exemple.

S'il s'agit au contraire d'une détection magnétique ou électromagnétique, ceci implique que chaque bille comporte une armature sphérique.

Dans d'autres modes de mise en oeuvre de l'invention qui peuvent s'appliquer selon les cas particuliers, le recyclage des billes selon le dispositif décrit peut se faire :
- automatiquement (détection du passage des billes par des cellules photoélectriques ou électromagnétiques) ;
- manuellement (impulsions électriques permettant le positionnement recherché du godet de la vanne motorisée) ;
- semi-automatique (dans le cas où les systèmes de détection peuvent s'avérer inopérants du fait par exemple de concentrations cellulaires trop élevées ou d'un diamètre de tubulure trop important). Dans ce cas, le positionnement adéquat du godet de la vanne motorisée est déterminé par deux temporisations. Une première temporisation détermine la durée pendant laquelle le godet reste en position renversée (ouverture vers le bas). Sa durée est fonction de la longueur de la tubulure à nettoyer et du débit de circulation de la culture. A la fin de cette temporisation, la position du godet est inversée. Une deuxième temporisation détermine la durée pendant laquelle le godet reste dans cette position ; elle a pour objet de permettre un bon positionnement de la bille dans le godet.

Le dispositif de nettoyage objet de l'invention :
- permet un recyclage automatique et en continu des billes dans les tubulures ;
- ne met pas de pompe en oeuvre et n'est donc pas dommageable vis-à-vis des cellules alguales ;
- n'implique ni d'arrêter la circulation de la culture, ni d'ouvrir le système et donc limite les risques de contaminations ;
- conduit à une proportion de culture réintroduite en même temps que la bille à l'entrée du récepteur solaire (et qui n'est donc pas carbonatée) très faible (volume inférieur à 1/1000 du volume totale de culture).

## Revendications

1. Photobioréacteur comportant un récepteur solaire (24) associé à un carbonateur (6) tous deux étant parcourus en série dans une boucle fermée (22) par une solution de milieu nutritif, un conduit de dérivation (26) étant prévu entre la sortie (28) et l'entrée (30) du récepteur solaire (24) pour permettre une circulation de billes de nettoyage (34) de la canalisation dans le seul récepteur solaire (24), caractérisé en ce qu'il comprend :
sur une portion verticale (26) du conduit de dérivation, une vanne motorisée (38) provoquant le passage automatique des billes (34), à l'exclusion de la solution ;
un manchon pivotant (42) intercalé dans la canalisation d'entrée (30) du récepteur solaire, en aval de l'embranchement avec le conduit de dérivation (26), ce manchon (42) servant de support à une vanne manuelle orientable (40) vis-à-vis de la pesanteur et permettant l'introduction ou le retrait des billes (34) de nettoyage dans/hors de la canalisation (22) ;
deux doigts de gant étanches équipés chacun d'une tige pénétrante réglable permettant de stopper ou de laisser passer les billes de nettoyage (34) en laissant circuler la solution, et situés ;
a) pour le premier (44), à l'embranchement de la canalisation d'entrée (36), du carbonateur et du conduit de dérivation (26) ;
b) pour le second (48), dans la canalisation d'entrée (30) du récepteur solaire, immédiatement en aval de la vanne manuelle orientable (40).

2. Photobioréacteur selon la revendication 1, caractérisé en ce que la vanne motorisée (38) et la vanne manuelle orientable (40) ont un obturateur sphérique (74) creusé d'un trou borgne (72) aux dimensions légèrement supérieures à celle d'une bille.

3. Photobioréacteur selon la revendication 2, caractérisé en ce que la vanne motorisée (38) est équipée d'un détecteur de présence de billes (5) qui, dès la chute par gravité d'une bille dans le trou borgne (72) tourné vers le haut en position d'attente, commande la rotation de l'obturateur (70) une première fois de 180° pour amener l'ouverture du trou borgne vers le bas et libérer la bille par gravité dans le conduit général (22) puis, une deuxième fois de 180°, pour remettre la vanne (38) en position d'attente de la bille suivante, le trou borgne (72) ouvert vers le haut.

4. Photobioréacteur selon la revendication 2, caractérisé en ce que le manchon pivotant (42) est placé dans une première position, amenant la vanne manuelle (40) en position verticale au-dessus de la canalisation d'entrée (30) dans le récepteur solaire (24), pour l'introduction d'une bille dans cette canalisation, et dans une deuxième position à 180° de la première, amenant la vanne manuelle (40) en position verticale au-dessous de la canalisation (30) d'entrée dans le récepteur solaire, pour l'extraction d'une bille hors de cette canalisation.

## Claims

1. Photobioreactor having a solar receptor (24) associated with a carbonator (6), both being traversed in series in a closed loop (22) by a nutrient medium solution, a branch duct (26) being provided between the outlet (28) and inlet (30) of the solar receptor (24) to permit a circulation of cleaning balls (34) from the pipe to the solar receptor (24), characterized in that it comprises:
- on a vertical portion (26) of the branch duct, a motorized valve (38) bringing about the automatic passage of the balls (34), with the exclusion of the solution;
- a pivoting sleeve (42) placed in the intake pipe (30) of the solar receptor, downstream of the junction with the branch duct (26), said sleeve (42) serving as a support for a manual valve (40) orientable with respect to gravity and permitting the introduction or removal of cleaning balls (34) with respect to the pipe (22);
- two tight glove fingers, each equipped with a regulatable penetrating rod able to stop or permit the passage of the cleaning balls (34) whilst allowing the circulation of the solution and positioned a) for the first (44), at the junction of the intake pipe (36), the carbonator and the branch duct (26), b) for the second (48), in the intake pipe (30) of the solar receptor, immediately downstream of the orientable manual valve (40).

2. Photobioreactor according to claim 1, characterized in that the motorized valve (38) and the orientable manual valve (40) have a spherical cap (74) with a blind hole (72) having dimensions slightly larger than those of a ball.

3. Photobioreactor according to claim 2, characterized in that the motorized valve (38) is equipped with a ball presence detector (5) which, as soon as a ball drops by gravity into the blind hole (72) turned upwards in the waiting position, controls the rotation of the cap (70) by 180° in order to bring the opening of the blind hole towards the bottom and thus release the ball by gravity into the general duct (22) and then a further 180° rotation in order to again bring the valve (38) into the waiting position for the following ball, with the blind hole (72) open towards the top.

4. Photobioreactor according to claim 2, characterized in that the pivoting sleeve (42) is placed in a first position bringing the manual valve (40) into a vertical position above the intake pipe (30) in the solar receptor (24), for the introduction of a ball into said pipe, and into a second position at 180° from the first, bringing the manual valve (40) into a vertical position above the intake pipe (30) in the solar receptor, for extracting a ball from said pipe.

## Patentansprüche

1. Photobioreaktor mit einem Solaremnfänger (24), verbunden mit einem Carbonateur (6), beide in Folge in einer geschlossen Schleife (22) durchflossen von einer Nährmittellösung, wobei eine Abzweigleitung (26) vorgesehen ist zwischen dem Ausgang (28) und dem Eingang (30) des Solarempfängers (24), um eine Zirkulation von Reinigungskugeln (34) der Leitung nur des Solarempfängers (24) zu ermöglichen,
**dadurch gekennzeichnet,** daß er umfaßt:
auf einem vertikalen Teil (26) der Abzweigleitung ein motorisiertes Ventil (38), das den automatischen Durchlauf der Kugeln (34) bewirkt, mit Ausschluß der Lösung;
einen drehbaren Stutzen (42), eingefügt in die Eingangsleitung (30) des Solarempfängers, flußabwärts von dem Anschluß der Abzweigleitung (26), wobei dieser Stutzen (42) als Stütze für ein manuell verstellbares Ventil (40) gegenüber der Schwerkraft dient und den Eintritt oder den Austritt der Reinigungskugeln (34) in/aus der Leitung (22) ermöglicht;
zwei dichte Handschuhfinger, jeder ausgestattet mit einer verstellbaren, eindringenden Stange, die ermöglicht, die Reinigungskugeln (34) anzuhalten oder durchzulassen durch Zirkulierenlassen der Lösung, und angeordnet,
a) den ersten (44) betreffend, an der Verzweigung der Eingangsleitung (36) des Carbonateurs und der Anzweigungsleitung (26);
b) den zweiten (48) betreffend, in der Eingangsleitung (30) des Solarempfängers, unmittelbar flußabwärts von dem manuell verstellbaren Ventil (40).

2. Photobioreaktor nach Anspruch 1, dadurch gekennzeichnet, daß das motorisierte Ventil (38) und das manuell verstellbare Ventil (40) einen Kugelverschluß (70) aufweisen, versehen mit einem Grundloch (72) mit etwas größeren Abmessungen als der einer Kugel.

3. Photobioreaktor nach Anspruch 2, dadurch gekennzeichnet, daß das motorisierte Ventil (38) mit einem Detektor (5) für das Vorhandensein von Kugeln ausgestattet ist, der im Moment des durch Schwerkraft bedingten Falls einer Kugel in das nach oben in die Wartestellung gedrehte Grundloch (72) ein erstes Mal die Drehung des Verschlusses (70) um 180° steuert, um die Öffnung des Grundloches nach unten zu bewegen und die Kugel mittels Schwerkraft in die Hauptleitung (22) freizugeben, und dann ein zweites Mal um 180°, um das Ventil (38) in Wartestellung für die folgende Kugel zu versetzen, wobei das Grundloch (72) nach oben offen ist.

4. Photobioreaktor nach Anspruch 2, dadurch gekennzeichnet, daß der drehbare Stutzen (42) in eine erste Stellung gebracht wird, die das manuelle Ventil (40) in Vertikalstellung oberhalb der Eingangsleitung (30) in den Solarempfänger (24) bringt, zur Einführung einer Kugel in diese Leitung, und in eine zweite, gegenüber der ersten um 180° gedrehte Stellung, die das manuelle Ventil (40) in eine Vertikalstellung unterhalb der Eingangsleitung (30) in den Solarempfänger bringt, zum Entleeren einer Kugel aus dieser Leitung.
